(19) Europäisches Patentamt
European Patent Office
Office européen des brevets

(11) **EP 2 250 961 A2**

(12) **EUROPEAN PATENT APPLICATION**

(43) Date of publication:
**17.11.2010 Bulletin 2010/46**

(51) Int Cl.:
***A61B 5/0452*** *(2006.01)*

(21) Application number: **10157346.7**

(22) Date of filing: **23.03.2010**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK SM TR**<br>Designated Extension States:<br>**AL BA ME RS** | (72) Inventors:<br>• **Moulder, J. Christopher**<br>  **Portland, OR 97219 (US)**<br>• **Whittington, R. Hollis**<br>  **Portland, OR 97202 (US)**<br>• **Müssig, Dirk**<br>  **West Linn, OR 97068 (US)** |
| (30) Priority: **13.05.2009 US 177671 P** | (74) Representative: **Lindner-Vogt, Karin L.**<br>**Biotronik SE & Co. KG**<br>**Woermannkehre 1**<br>**12359 Berlin (DE)** |
| (71) Applicant: **Biotronik CRM Patent AG**<br>**6341 Baar (CH)** | |

(54) **System, method and computer-readable storage medium for heart signal detection**

(57)     The invention is related to the detection of electrical signals originating from a human or animal heart. In particular for monitoring devices it is desired to obtain electrical signals from a human or animal heart with electrical contacts at the body of an implantable medical device, hence without the need to implant electrical leads to the hearts.

Hence, a method, a system and a computer-readable storage medium for detecting electrical signals originating from a human or animal heart is proposed. The method comprising the following steps:
- receiving electrical signals in at least two sensing channels,
- combining the electrical signals for forming a combined channel,
- extracting a template from the signals of the combined channel,
- comparing incoming electrical signals with the template, and
- depending from the result of the comparison, performing at least one of controlling one or more devices and signalling the result.

FIG. 2

**Description**

The invention is related to the detection of electrical signals originating from a human or animal heart. Such signals can be obtained from electrodes implanted in the heart tissue or from electrical contacts at the body of an implantable medical device. Typically, these electrical signals are processed to identify contractions of the heart tissue and are used to control electrical stimulation devices. Alternatively the electrical signals are recorded within the implanted medical device and/or transmitted outside the body to external devices, remote databases, expert systems or the like for further evaluation. The implantable medical device can be an implantable pacemaker, cardioverter-defibrillator, or a monitoring device.

In particular for monitoring devices it is desired to obtain electrical signals from a human or animal heart with electrical contacts at the body of an implantable medical device, hence without the need to implant electrical leads to the hearts. One drawback of such systems is to the decreased ability to correctly identify weak signals in the presence of increased noise.

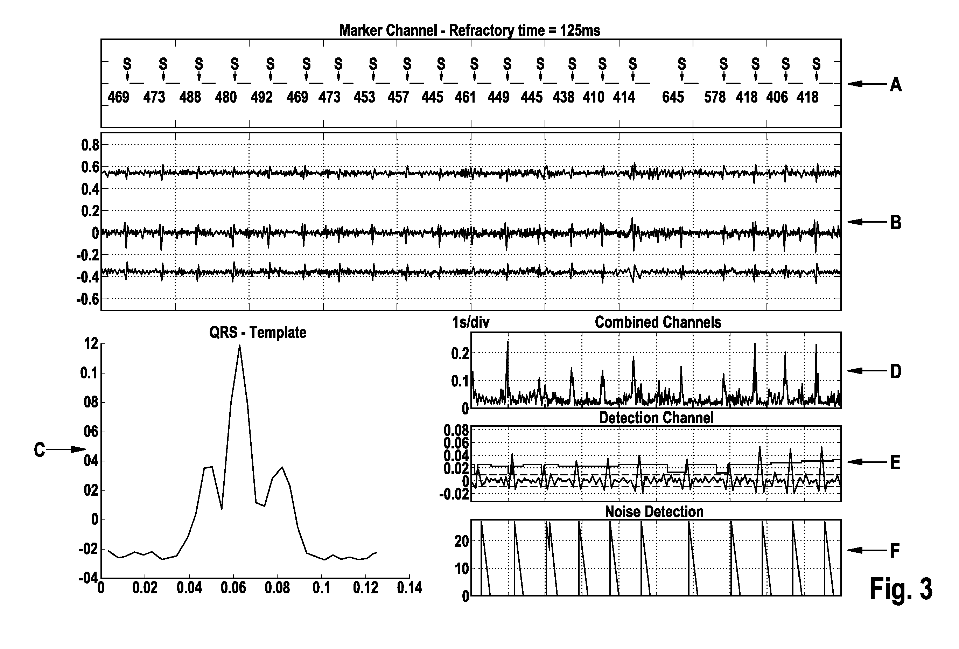

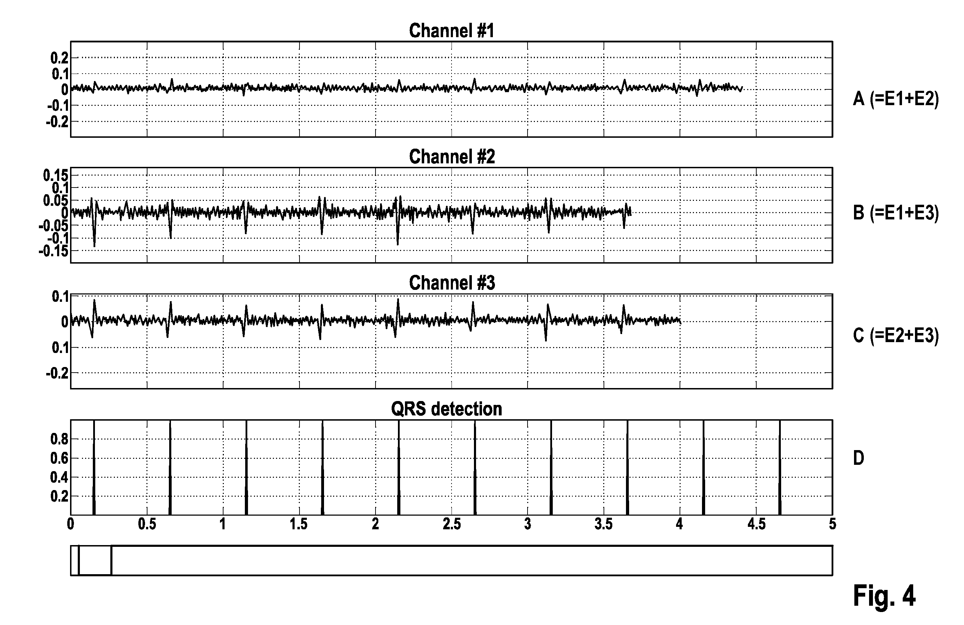

Implantable monitoring devices that record and monitor electrical activity of the heart known in the art typically use two sensing electrodes. While they are smaller, signal quality is known to be poor. A single vector, whether on a short lead or integrated into the device, is highly susceptible to spurious noise and loss of sensitivity, for example due to slight electrode orientation changes by movement of the implanted device within the body. Implantation of these devices is also time consuming since vector mapping must be done to ensure the device is implanted in an orientation to produce the best signal. Figures 3(B) & 4(A, B, C) show recorded subcutaneous electrograms (SEGM) from three vectors. Notice that the signal is lost in the top channel or has a very low signal to noise ratio (SNR). With the random loss of signal or increased noise, false detections or loss of detections can cause subsequent algorithms, such as atrial arrhythmia detection, to fail.

Presently, typical signal characteristics of the signals originating from heart activity like QRS complexes are detected by comparing the input signal amplitude to a dynamic sensing threshold. The threshold is set to a percentage of the previous detected maximum amplitude. Noise is detected when the signal crosses a target threshold and activates a noise counter. The noise counter can be re-activated every time the signal crosses the threshold while the counter is active. While the counter is active, no new QRS detections are allowed. In each case (sensing and noise detection) the absolute value of the signal is used to detect large positive or negative spikes, since the polarity of the QRS complex is not known. When the signal to noise ratio (in this case, amplitude of QRS complex to any other signal between those complexes) is low, the probability of oversensing (detecting noise as valid QRS events) or undersensing (missing QRS events) is greatly increased.

In US 6,699,200 B2 an implantable medical device with multi-vector sensing electrodes is described. It is proposed monitoring different channels each originating from a pair of electrodes and capturing the maximum signal for appropriate sensing. In cases where the signal amplitude from one electrode pair drops dramatically, it is switched to another pair of electrodes.

The present invention is directed to a method to obtain electrical signals of a human or animal heart by an implantable device with sensing electrode contacts thereon, hence without the need for implanted electrodes at the heart tissue. In this case the amplitude of noise in relation to the signal amplitude is increased. This is the result of at least two factors, the distance between electrodes is small, thereby reducing electrocardiogram (ECG) amplitude and the proximity to muscle tissue increases EMG (electromyogram) signals. ECG noise is overlapping the frequency range of the ECG so simple filtering does not always reduce it. The invention is therefore further directed to a sensing algorithm that increases signal-to-noise ratio and improves QRS detection from weak signals in the presence of increased noise.

To overcome the disadvantages of former solutions, the invention proposes a method for detecting electrical signals originating from a human or animal heart where electrical signals of at least two sensing channels are evaluated. In a preferred embodiment three electrodes are connected to three sensing channels. However in other embodiments more than three electrodes may be connected to more than three sensing channels.

According to the invention the electrical signals received by the at least two sensing channels are combined to a single channel, a so called combined channel. Preferably, the electrical signals pass through a filter sensing block, before they are combined. In a preferred embodiment the electrical signals are combined by summing the weighted signals of the at least two sensing channels.

According to the invention from the incoming electrical signals of the combined channel at least a template is extracted. The time window for the template depends from the event to be detected. In a preferred embodiment a template of a QRS complex is extracted. For encompassing the entire QRS complex the width of the template is approximately 125ms. For extracting the template from the incoming data stream of the combined channel different suitable methods may be used, for example pattern recognition or simple threshold methods; for extracting a QRS complex in a preferred embodiment simple threshold methods are used. Extracted templates then are stored for further use.

At least a part of the extracted templates then is compared with the incoming electrical signals to detect signal patterns indicating an event such as for example abnormalities in the heart activities like contractions of the heart tissue.

Templates are compared preferably with the electrical signals of the combined sensing channel. Prior to performing the comparison in a preferred embodiment of the invention detection thresholds are calculated. At least one noise threshold is also defined. Detection thresholds and/or noise thresholds can be derived for example based on the extracted template.

In a preferred embodiment comparison of the incoming electrical signals with the at least one template comprises performing a pseudo cross covariance between the template(s) and the incoming data stream, which can be an ECG stream for example. From the calculated cross covariance signal and, if necessary, considering the thresholds, patterns are detected. In a preferred embodiment QRS complexes are detected. In the case of QRS complexes in another preferred embodiment detection is only performed on signals with positive values.

If patterns have been detected, electrical devices like a pacemaker, a cardioverter-defibrillator, a monitoring device or such may be controlled depending from the detected pattern, and/or the electrical signals are recorded within the implanted medical device and/or transmitted outside the body to external devices, remote databases, expert systems or the like for further evaluation.

In a further preferred embodiment detected patterns are combined with the template for obtaining a new template which reflects for example changes in the ECG.

In another preferred embodiment thresholds are adjusted, if within a predefined period of time no pattern has been detected. Preferably then the threshold is decreased.

A system for detecting electrical signals originating from a human or animal heart according to the invention comprises at least one data processing device. The system is configured in such a way that the following steps may be performed:

- receiving electrical signals in at least two sensing channels,
- combining the electrical signals for forming a combined channel,
- extracting a template from the signals of the combined channel,
- comparing incoming electrical signals with the template, and
- depending from the result of the comparison, performing at least one of controlling one or more devices and signalling the result.

In a preferred embodiment of the invention the device comprises at least three sensing electrodes which are connected to at least two sensing channels. Preferably the combination of the sensing electrodes with the sensing channels can be changed. In a preferred embodiment a switch matrix is used for changing the assignment of the electrodes to the sensing channels.

In a preferred embodiment the inventive system comprises an implantable device, which is realized for example as pacemaker, cardioverter-defibrillator, or a monitoring device. The system can also comprise one or more external devices such as remote databases, expert systems or the like.

An implantable device has in a preferred embodiment an enclosure made from an electrically conductive and biocompatible material like titanium for example. In another preferred embodiment the implantable device is made of a conductive body covered with a non-conductive material. Preferably, the non-conductive material has one or more holes that allow the conductive body to contact the surrounding tissue.

It is further an object of the invention to provide a computer-readable storage medium storing program code for causing a data processing device to perform a method for detecting electrical signals originating from a human or animal heart, the method comprising the steps of:

- receiving electrical signals in at least two sensing channels,
- combining the electrical signals for forming a combined channel,
- extracting a template from the signals of the combined channel,
- comparing incoming electrical signals with the template, and
- depending from the result of the comparison, performing at least one of controlling one or more devices and signalling the result.

The implantable medical device described herein uses electrodes to sense subcutaneous electrograms (SEGM). In one embodiment, the enclosure of the implantable medical device is of an electrically conductive and biocompatible material like titanium and comprises three or more isolated, electrically conductive electrode contacts forming electrodes. In another embodiment, electrode contacts are integrated in a part of the implantable medical device that is made of non-conductive material. In yet another embodiment, the conductive body is covered by a non-conductive material having one or more holes that allow the body to contact the tissue. It is to be understood that these embodiments can be combined in any suitable way.

The electrodes are connected to at least three sensing channels. In one embodiment with three sensing channels (A, B, C) and three electrodes (E1, E2, E3) sensing channel A is connected to electrodes E1 and E2, sensing channel B is connected to electrodes E1 and E3 and sensing channel C is connected to electrodes E2 and E3. It is to be understood that any combination of connecting electrodes to the sensing channels would be possible and that also more electrodes including the enclosure and also more sensing channels can be implemented. In an alternative embodiment the electrodes are connected to the sensing channels by a switch matrix that allows connecting any electrode contact to any sensing channel. In each sensing channel, the electrical signals pass through a filter sensing block for each channel. In one embodiment, the signals are digitized by an analog-to-digital converter as known in the art. It is to be understood that the further processing of the digitized signals could be performed by a microprocessor or programmable microcontroller or the like as known in the art.

The present invention seeks to first reduce noise levels by using an algorithm that relies on both the shape and amplitude of the QRS complex. Next the algorithm uses inherent properties to increase the detection SNR while not compromising noise detection.

Figure 1    shows a depiction of the heart in relation to an implanted medical device ac- cording to a preferred embodiment of the invention;

Figure 2    is a flow diagram of a preferred signal processing algorithm;

Figure 3    displays results using the preferred signal processing algorithm;

Figure 4    shows an example of five seconds of the three sensed (A, B, C) channels and the corresponding QRS detections;

Figure 5    shows the Gaussian distribution of the mean SNR in dB;

Figure 6    shows a table with exemplary values for different variables used in the signal processing algorithm;

Figure 7    shows a close-up of the derived cross-covariance signal used for detection of the QRS complexes.

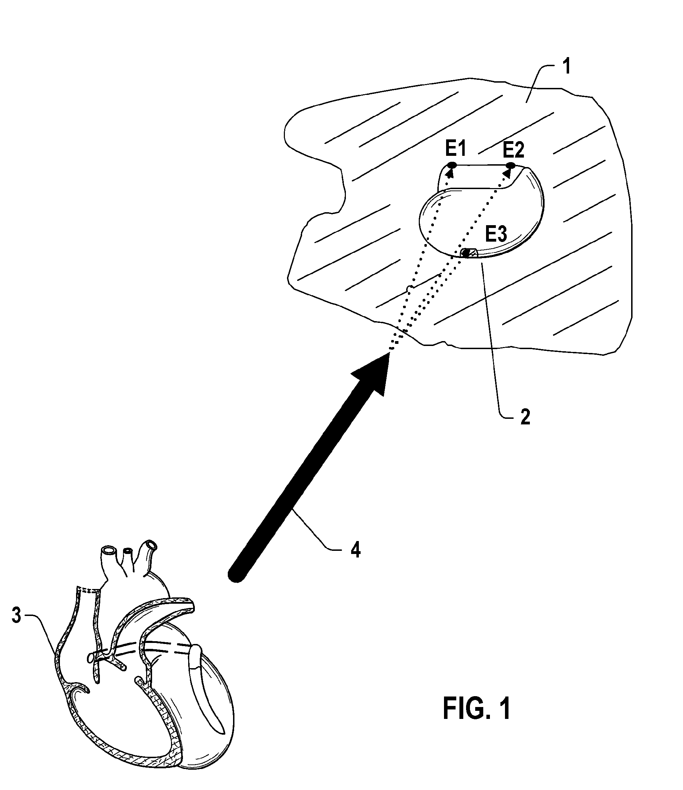

Figure 1 shows a depiction of the heart in relation to the implanted medical device according to the invention. (1) depicts the near field noise sources, such as muscle activity or rubbing of the electrodes. (2) shows the implanted device with three sensing electrodes. (3) shows the heart as a far field source; and (4) represents the electrical signal from the heart that is sensed at the three electrodes.

In a preferred embodiment three electrodes are arranged in a triangle to form three sensing channels. Any one electrode is used in two sensing channels. In one exemplary embodiment three sensing channels (A, B, C) and three electrodes (E1, E2, E3) are connected such that sensing channel A is connected to electrodes E1 and E3, sensing channel B is connected to electrodes E2 and E3 and sensing channel C is connected to electrodes E1 and E2. In general, near field-noise is sensed on all channels but in different polarities, amplitudes and phases. EGMs from the heart are sensed as far field signals and are always coincident on all channels, but have varying polarities and amplitudes based on electrode spacing and orientation to the heart.

SNR is increased in the following manner:

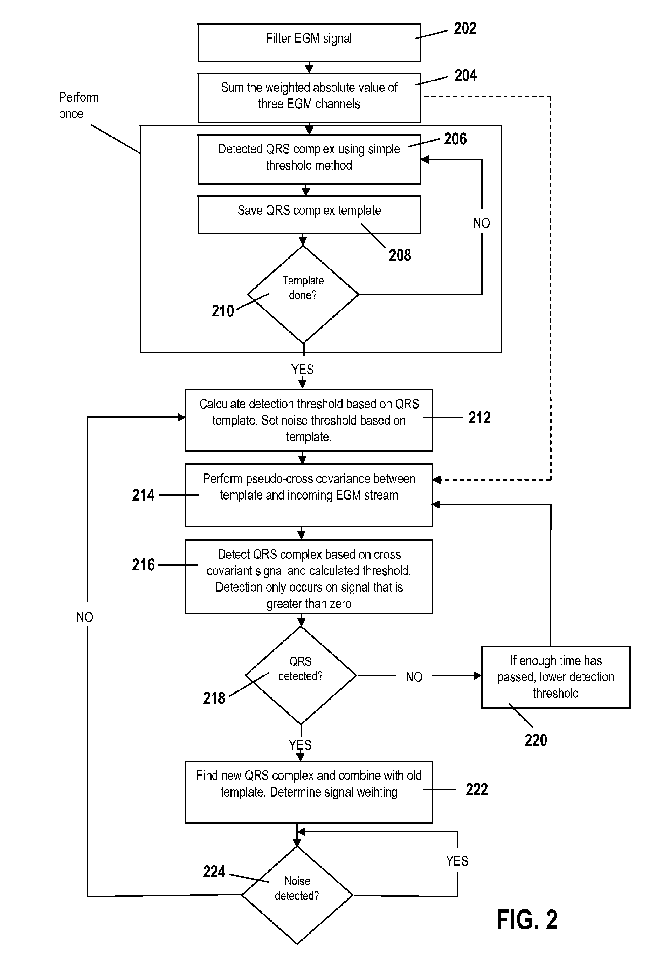

Figure 2 shows a flowchart of the signal processing algorithm. First, in step 202, electrical signals of the three sensing channels pass through a filter sensing block. However, it is also possible to use at least two channels. Then, a weighted sum of the absolute value of each of the three input channels is made in step 204 to form a combined channel. A template is made in step 206 from the combined QRS complex by means of standard detection methods. In a preferred embodiment, the template is approximately 125ms wide to encompass the entire QRS complex. The mean value of the template is subtracted so that there is no DC offset. Then the QRS complex is saved in step 208 for example by storing the template in storage means of the implanted device itself and/or in an external device belonging to the system for heart signal detection. After the QRS template is made, which is checked in step 210, the initial template making part comprising the steps 206 and 208 of the signal processing algorithm is finished.

In step 212 detection threshold is calculated and noise threshold is set based on the QRS template. The template is then cross-correlated in step 214 with the combined data channel to form a pseudo-cross-covariance (non-normalized). The resultant waveform reflects how similar the template and the combined data are. QRS complexes are detected in step 216 from this derived (cross-covariant) data stream. Cross-covariance significantly smoothes the combined data and increases SNR (by -8dB) while also transforming the signal from unipolar to bipolar. After each QRS detection, new complexes are extracted from the combined data. In step 222 the new complexes are then averaged into the current template, thus modifying it. This modification allows the template to maintain validity during changing signal conditions (i.e. device rotation due to postural changes). If, in step 218 it is recognized that no QRS structure is detected, the detection threshold is lowered in step 220 after enough time has elapsed. In step 224 noise detection is performed by a noise detection timer, if noise is detected, i.e. if the noise detection timer is active, no QRS detection can be performed.

In a preferred embodiment the non-normalized cross-correlation is calculated according to the equation (1):

$$R_{xy}(d) = \sum_i x_i \cdot y_i \ , \qquad\qquad (1)$$

cross-covariance is calculated according to equation (2):

$$\phi_{xy}(d) = \sum_i (x_i - \mu_x) \cdot (y_{i+d} - \mu_y) \ , \qquad\qquad (2)$$

and the proposed pseudo-cross-covariance according to equation (3):

$$c_{xy}(d) = \sum_i (x_i - \mu_x) \cdot y_{i+d} \ , \qquad\qquad (3)$$

where x represents the template, y represents the incoming combined channel data stream, $\mu$ represents the mean of the data set over indices i.

The calculations presented do not necessarily need to be done on all samples in the data stream and template. For example, every other point could be used. In an exemplary embodiment the incoming signals are sampled at 512Hz, yielding 64 data samples in the template for 125ms. It is possible to use only 32 of those samples for calculation to reduce the needed calculations. Similarly, every 4th sample could be used to yield only 16 operations. However, improvement in SNR and detection accuracy degrades as fewer samples are used.

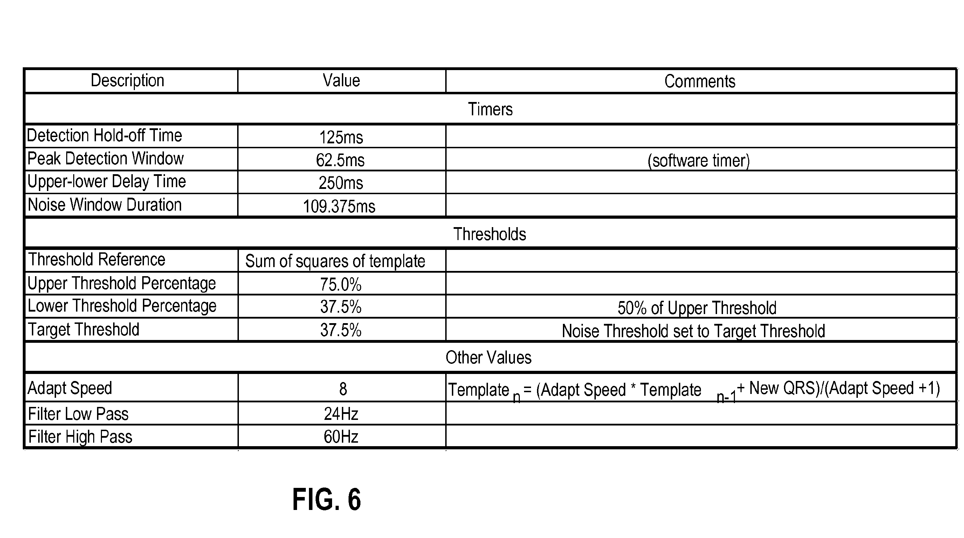

Figure 6 shows a table with exemplary values for different variables used in the signal processing algorithm.

Because the combined data and the template are both positively oriented, the region of interest in the cross-covariance between them is also positive. Therefore, QRS detection need only be done on positive values. Ignoring negative values in the cross-covariant signal further increases SNR (to ~+10dB). Noise detection can be performed on both positive and negative (or absolute value) or on positive only values.

In an alternative embodiment, the detection and noise thresholds can be dynamically altered based on the template. The invention uses the sum of the squares of the data points of the template to generate a value representing "signal quality." Detection and noise thresholds are set based on this value. The resultant threshold is a measure of the fit between the template and the combined data and not simply the amplitude of the previously detected QRS peak.

Figure 3 displays results using the signal processing algorithm on data obtained from an animal with an implanted medical device according to the present invention. (A) shows detection markers and the time between detections in milliseconds. (B) shows the raw EGM signal from each channel. (C) displays the template used for cross-correlation. (D) shows the combined data after weighted summation. (E) shows the results of cross-correlation. Finally, (F) shows the noise detection timer (when active, no detections can take place).

Figure 4 shows five seconds of the three sensed (A, B, C) channels and the corresponding QRS detections (D). Channel #1 (A) has a very low SNR and is unsuitable to be used for detection. Channels #2 and #3 have much larger QRS complexes; however, the signal and noise amplitudes vary widely per beat.

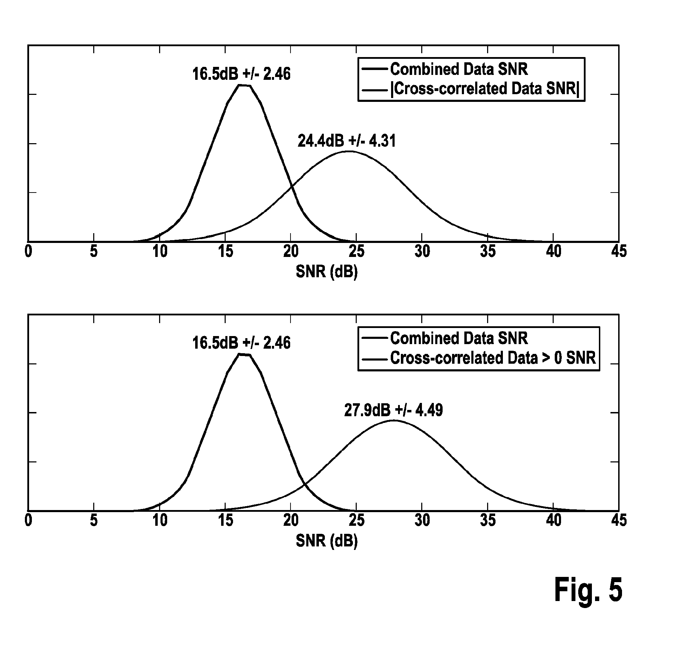

Figure 5 shows the Gaussian distribution of the mean SNR in dB. The beat-beat SNR is defined as the ratio between the mean peak signal amplitude (QRS peak) of two consecutive peaks (beat-beat) and the mean amplitude of the signal between those peaks. The mean SNR is the average of all the beat-beat SNR over the recorded file. The upper part of Figure 5 (A) uses the absolute value of the signal between peak detections for the derived channel and the lower part of Figure 5 (B) uses only the values greater than zero. Both distributions are presented since the detection algorithm detects QRS complexes using only the positive values and detects noise using the absolute value. The method of detection denoted by (A) should have a slightly increased specificity and decreased sensitivity relative to the method in (B).

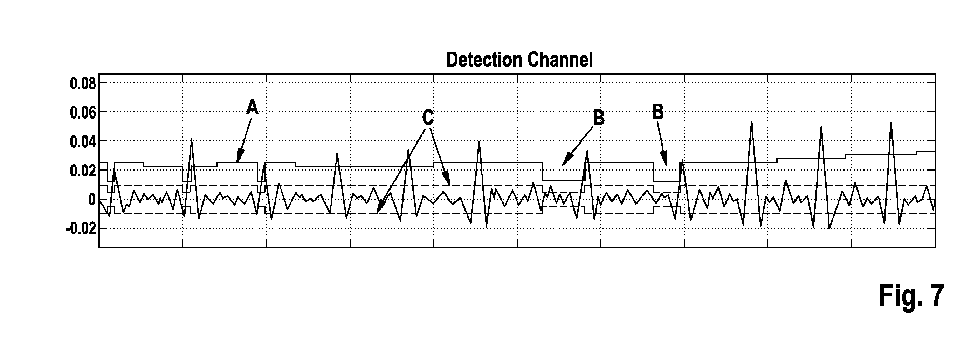

Figure 7 shows a close-up of the derived cross-covariance signal used for detection of the QRS complexes. (A) shows a line that denotes the QRS detection threshold. (B) indicates when the upper-lower delay timer expires and the sensing threshold is lowered. (C) shows the positive and negative noise sensing thresholds (dashed lines). Noise detection is only started after a QRS peak is detected.

**LIST OF ABBREVIATIONS:**

SEGM    subcutaneous electrogram

SNR     signal to noise ratio

QRS     signal representing ventricular depolarization of the heart

ECG     electrocardiogram

EMG     electromyogram

**Claims**

1.  A method for detecting electrical signals originating from a heart, comprising the following steps:

    receiving electrical signals in at least two sensing channels,
    combining the electrical signals for forming a combined channel,
    extracting a template from the signals of the combined channel,
    comparing incoming electrical signals with the template, and
    depending from the result of the comparison, performing at least one of controlling one or more devices and signalling the result.

2.  The method according to claim 1, where combining the electrical signals comprises summing weighted values of the at least two sensing channels.

3.  The method according to claim 1, where the step of comparing comprises detecting patterns of signals.

4.  The method according to claim 3, where the step of comparing comprises performing a cross-correlation between the template and the incoming electrical signals.

5.  The method according to claim 3, where detecting the patterns of signals is performed with respect to a threshold.

6.  The method according to claim 5, where the threshold is adapted if during a predefined period of time no pattern has been detected.

7.  The method according to claim 1, where detected patterns of signals are combined with the template.

8.  A system for detecting electrical signals originating from a heart, the device comprising at least one means for data processing and being arranged in such a manner that a method according to claim 1 is executable.

9.  The system according to claim 8, where the system comprises at least three electrodes.

10. The system according to claim 9, where the at least three electrodes are connected by a switch matrix to sensing channels.

11. The system according to claim 8, where the system comprises at least one of an implantable device and an external device.

12. The system according to claim 11, where the implantable device comprises an enclosure of electrically conductive or of electrically non-conductive material.

13. The system according to claim 12, where the electrically non-conductive material covers a electrically conductive body, the electrically non-conductive enclosure having one or more holes that allow the conductive body to contact surrounding tissue.

14. The system according to claim 11, where the implantable device is a pacemaker, cardioverter-defibrillator, or a monitoring device

15. A computer-readable storage medium storing program code for causing a data processing device to perform a method according to claim 1.

FIG. 1

FIG. 2

Marker Channel - Refractory time = 125ms

Combined Channels

Detection Channel

Noise Detection

1s/div

QRS - Template

Fig. 3

Channel #1

A (=E1+E2)

Channel #2

B (=E1+E3)

Channel #3

C (=E2+E3)

QRS detection

D

**Fig. 4**

EP 2 250 961 A2

Fig. 5

| Description | Value | Comments |
|---|---|---|
| Timers | | |
| Detection Hold-off Time | 125ms | |
| Peak Detection Window | 62.5ms | (software timer) |
| Upper-lower Delay Time | 250ms | |
| Noise Window Duration | 109.375ms | |
| Thresholds | | |
| Threshold Reference | Sum of squares of template | |
| Upper Threshold Percentage | 75.0% | |
| Lower Threshold Percentage | 37.5% | 50% of Upper Threshold |
| Target Threshold | 37.5% | Noise Threshold set to Target Threshold |
| Other Values | | |
| Adapt Speed | 8 | $Template_n = (Adapt\ Speed * Template_{n-1} + New\ QRS)/(Adapt\ Speed +1)$ |
| Filter Low Pass | 24Hz | |
| Filter High Pass | 60Hz | |

**FIG. 6**

EP 2 250 961 A2

Detection Channel

Fig. 7

EP 2 250 961 A2

**REFERENCES CITED IN THE DESCRIPTION**

**Patent documents cited in the description**

- US 6699200 B2